# FASCICULE DE BREVET EUROPEEN

(11) **EP 2 167 455 B1**
(45) Date de publication et mention de la délivrance du brevet: **12.11.2014**
(21) Numéro de dépôt: 08785983.1
(22) Date de dépôt: 08.07.2008
(51) Int. Cl.: C07C 201/08, C07C 201/16, C07C 205/06, B01D 17/02

(54) **PROCEDE DE PREPARATION DE DINITROTOLUENE**
VERFAHREN ZUR HERSTELLUNG VON DINITROTOLUOL
PROCESS FOR PREPARING DINITROTOLUENE

(30) Priorité: 19.07.2007 FR 0705231
(43) Date de publication de la demande: 31.03.2010
(73) Titulaire: Vencorex France, 69800 Saint-Priest (FR)
(72) Inventeur: MARION, Philippe, F-69390 Vernaison (FR)
(74) Mandataire: Colombet, Alain André
(86) Numéro de dépôt international: PCT/EP2008/058870
(87) Numéro de publication internationale: WO 2009/010426

(56) Documents cités:
- EP-A- 0 903 336

## Description

La présente invention a pour objet un procédé de préparation de dinitrotoluène.

Plus précisément, l'invention vise un procédé perfectionné de préparation de dinitrotoluène à partir du mononitrotoluène.

Le dinitrotoluène est un produit industriel important car il est l'intermédiaire de fabrication du toluènediisocyanate.

Le dinitrotoluène est obtenu par double nitration du toluène. Les opérations de nitration n'étant pas sélectives, le dinitrotoluène est en fait un mélange d'isomères de 2,4-dinitrotoluène et de 2,6-dinitrotoluène associé à différentes impuretés qui sont d'autres isomères tels que : 2,3-dinitrotoluène, 2,5-dinitrotoluène, 3,4-dinitrotoluène et 3,5-dinitrotoluène. Dans le mélange, le ratio entre le 2,4-dinitrotoluène et le 2,6-dinitrotoluène est égal à 4 ± 0,3 et la teneur en impuretés est généralement comprise entre 3 et 4,5 % en poids.

Dans EP-A 0 903 336, il est décrit un procédé de préparation de dinitrotoluène par double nitration du toluène à l'aide d'un mélange d'acide nitrique et d'acide sulfurique avec la caractéristique de faire appel à de l'acide sulfurique dilué.

Classiquement, la production du dinitrotoluène s'effectue selon un procédé en deux étapes comme décrit notamment dans l'encyclopédie KIRK-OTHMER, Encyclopedia of Chemical Technology, 15, p 927 et suivantes (3^{ème} édition) et par H. Hermann et al dans l'ouvrage Nitration, Recent Laboratory and Industrial Developments, (Chapitre 21, Lyle F. Albright , Richard V. C. Carr, Robert J. Schmitt, American Chemical Society, Washington DC 1996).

La première étape consiste à effectuer la nitration du toluène avec l'acide nitrique, en présence d'acide sulfurique et la séparation du mononitrotoluène obtenu à partir du mélange réactionnel puis suit une deuxième étape de nitration du mononitrotoluène obtenu à l'aide d'acide nitrique et en présence d'acide sulfurique suivie par la séparation du dinitrotoluène obtenu.

On effectue donc dans une première étape, la nitration du toluène à l'aide d'un mélange nitrant dont la composition peut varier et comprendre par exemple, de 50 à 60 % en poids d'acide sulfurique, de 15 à 40 % d'acide nitrique et de 10 à 20 % en poids d'eau.

La quantité d'acide nitrique mise en oeuvre est généralement supérieure à la quantité requise par la stoechiométrie de la réaction de nitration : le ratio molaire acide nitrique/toluène étant compris entre 1,05 et 1,2.

Après achèvement de la réaction, il y a séparation d'une phase aqueuse comprenant les acides résiduaires (acide sulfurique, acide nitrique) avec majoritairement de l'acide sulfurique et une phase organique comprenant majoritairement le mononitrotoluène.

On effectue dans une étape suivante la nitration du mononitrotoluène compris dans la phase organique à l'aide d'un mélange nitrant.

D'une manière générale, on a intérêt pour des raisons de productivité et de dimensionnement d'appareillage, de partir d'un mélange nitrant à faible teneur en eau. Ainsi, on fait appel préférentiellement à un mélange comprenant de 55 à 70 % en poids d'acide sulfurique, de 20 à 44,5 % d'acide nitrique et de 0,5 à 10 % en poids d'eau.

La quantité d'acide nitrique mise en oeuvre dans cette réaction de nitration est supérieure à la quantité requise par la stoechiométrie de la réaction : le ratio molaire acide nitrique/mononitrotoluène étant compris entre 1,05 et 1,2.

En fin de réaction, on obtient une phase aqueuse et une phase organique qu'il faut séparer.

Dans les conditions d'un flux nitrant concentré en acides, le ratio pondéral, en sortie de réaction de dinitration, entre la phase aqueuse et la phase organique est inférieur à 1 et se situe plus particulièrement entre 0,75 et 0,95 ce qui signifie que l'on obtient très préférentiellement une phase aqueuse dispersée dans la phase organique.

Comme mentionné précédemment, il importe de mettre en oeuvre un mélange nitrant concentré en acides nitrique et sulfurique.

Toutefois, dans ces conditions, on a constaté que la séparation des phases organique et aqueuse effectuée selon la technique de séparation par décantation était laborieuse. Ainsi, des durées de 48 heures étaient nécessaires pour effectuer la séparation.

Par ailleurs, il a été montré que la technique de séparation s'avérant difficile, il n'était pas possible de diminuer la quantité d'acide nitrique excédentaire mise en oeuvre lors de la deuxième opération de nitration car dans ce cas, la séparation était rendue encore plus difficile même dans le cas où la séparation était effectuée par centrifugation.

L'objectif de la présente invention est de fournir un procédé perfectionné de nitration du mononitrotoluène.

L'un des buts à atteindre par l'invention est de mettre à disposition un procédé permettant une séparation plus aisée des phases aqueuse et organique suite à l'opération de nitration du mononitrotoluène.

Un autre but de l'invention est de permettre une baisse de la quantité d'acide nitrique excédentaire mise en oeuvre dans la deuxième étape de nitration et ainsi de réduire la quantité d'acide nitrique présente dans le dinitrotoluène envoyé ensuite à la purification par lavage : l'acide nitrique excédentaire sera alors retrouvé dans les effluents aqueux du procédé éventuellement recyclés ou traités.

Conformément à la présente invention, il a été trouvé que l'on pouvait faciliter la séparation des phases aqueuse et organique et baisser la quantité d'acide nitrique excédentaire dès lors que l'on effectuait avant la séparation des phases, une inversion de phase c'est-à-dire que l'on dispersait la phase organique dans la phase aqueuse.

Ce changement de phase continue s'opère en modifiant le ratio entre la phase aqueuse et la phase organique et en choisissant le ratio pondéral entre la phase aqueuse et la phase organique supérieur à 1,2, de préférence compris entre 1,5 et 3,5, et encore plus préférentiellement compris entre 1,8 et 3,0.

Plus précisément, l'objet de la présente invention est un procédé de préparation de dinitrotoluène à partir du mononitrotoluène qui comprend la réaction de nitration du mononitrotoluène à l'aide d'un mélange nitrant comprenant de l'acide nitrique, de l'acide sulfurique et de l'eau conduisant à un milieu bi-phasique puis la séparation des phases organique et aqueuse dudit milieu bi-phasique qui est caractérisé par le fait que l'on effectue la nitration du mononitrotoluène à l'aide d'un mélange nitrant comprenant au plus 10 % en poids d'eau conduisant à un milieu bi-phasique, que l'on sépare les phases organique et aqueuse dudit milieu bi-phasique, et que l'on recycle la phase aqueuse issue de l'opération de séparation, en fin de réaction de nitration du mononitrotolène et avant la séparation des phases organique et aqueuse de telle sorte que le rapport pondéral entre la phase aqueuse et la phase organique soit au moins égal à 1,2.

Ainsi, selon le procédé de l'invention, on ajuste le ratio des phases aqueuse et organique par recyclage de la phase aqueuse comprenant majoritairement de l'acide sulfurique et qui est récupérée suite à la séparation des phases organique et aqueuse.

L'introduction d'un flux aqueux d'acide sulfurique est effectuée en amont de l'opération de séparation des phases aqueuse et organique et permet d'effectuer un changement de phase continue et ainsi une meilleure séparation des phases aqueuse et organique puisque le temps de décantation devient inférieur à 1 minute.

De plus, il devient alors possible de réduire l'excès d'acide nitrique mis en oeuvre, sans dégrader l'opération de nitration de l'invention. On précise à titre d'exemple que la quantité d'acide nitrique peut être réduite de 50 à 75 % en poids.

Il est à noter que l'introduction de la phase aqueuse acide est effectuée dans le milieu bi-phasique résultant de la nitration du mononitrotoluène.

Toutefois, le point d'introduction peut avoir lieu lorsque le taux de transformation du nitrotoluène est égal ou proche de 100 % mais également lorsqu'il est inférieur mais de préférence d'au moins 90 %.

Par « fin de réaction de nitration », on entend un taux de transformation du nitrotoluène d'au moins 90 %.

Par taux de transformation (TT), on entend le rapport entre le nombre de moles de nitrotoluène transformées et le nombre de moles de nitrotoluène engagées.

En effet, il est courant d'effectuer les opérations de nitration dans une cascade de réacteurs, en particulier dans une suite d'au moins deux réacteurs, le (ou les premiers) dénommés « réacteurs de nitration » permettant d'effectuer la nitration du mononitrotoluène avec un taux de transformation d'au moins 90 % associé à un deuxième type de réacteur dit « réacteur de finition » qui permet de parfaire le taux de transformation jusqu'à un taux égal ou proche de 100 %.

Le réacteur de finition se distingue des précédents par le fait qu'il ne comporte pas d'introduction de mélange d'acides nitrant.

Ainsi, l'invention inclut le cas où la phase aqueuse acide séparée est ajoutée, en fin de réaction de nitration, en aval du ou des réacteurs de nitration et en amont ou au niveau du réacteur de finition.

Conformément au procédé de l'invention, on part d'une solution de nitrotoluène qui peut être préparée d'une manière classique selon les techniques décrites dans la littérature.

Selon un mode de réalisation préféré, on effectue dans une première étape, la nitration du toluène avec l'acide nitrique, en présence d'acide sulfurique ce qui conduit à un milieu biphasique qui est soumis ensuite à une séparation afin d'obtenir d'une part une phase aqueuse et d'autre part une phase organique comprenant majoritairement du mononitrotoluène qui sera ensuite soumise à une opération subséquente de nitration.

La première opération de nitration peut être conduite en discontinu (batch) mais l'on préfère le mode continu.

Selon un procédé discontinu, le toluène est introduit dans un réacteur refroidi (environ 25°C) puis sa nitration est effectué à l'aide d'un mélange générateur d'ions nitronium NO₂⁺ dont la composition peut varier et comprendre par exemple, de 50 à 60 % en poids d'acide sulfurique, de 15 à 40 % d'acide nitrique et de 10 à 20 % en poids d'eau.

La réaction de nitration doit être conduite dans un réacteur présentant de bonnes performances en terme de transfert de matière et de transfert thermique.

On peut utiliser par exemple le réacteur boucle de Meissner où l'agitation est effectuée par un circulateur ou le réacteur de Biazzi qui est une cuve agitée.

La quantité d'acide nitrique mise en oeuvre est supérieure à la quantité requise par la stoechiométrie de la réaction. Le ratio molaire acide nitrique/toluène est compris entre 1,05 et 1,2.

Ledit mélange est ajouté progressivement dans le toluène tout en maintenant le mélange réactionnel à 25°C.

Après l'addition du mélange nitrant, la température remonte entre 35 et 55°C.

La réaction de mononitration du toluène est généralement mise en oeuvre sous pression atmosphérique bien que des pressions plus élevées peuvent être également utilisées.

Selon une variante préférée du procédé de l'invention, on conduit cette étape de nitration sous atmosphère contrôlée de gaz inertes afin d'être dans des conditions de concentration des produits gazeux dans le ciel en dehors de la zone d'inflammabilité. On peut établir une atmosphère de gaz rares, de préférence l'argon mais il est plus économique de faire appel à l'azote.

Après achèvement de la réaction, il y a séparation d'une phase organique comprenant majoritairement le mononitrotoluène et une phase aqueuse comprenant les acides résiduaires avec majoritairement de l'acide sulfurique.

La séparation peut être effectuée selon les techniques classiques de séparation liquide/liquide telles que la centrifugation ou la décantation statique.

Selon un mode de réalisation en continu, on commence généralement à charger un pied de cuve comprenant une solution aqueuse d'acide sulfurique, à savoir une solution ayant une concentration d'acide sulfurique variant par exemple entre 65 et 80 % en poids.

On alimente en parallèle le toluène et le mélange d'acide nitrique et d'acide sulfurique de telle sorte que le ratio acide nitrique/ toluène soit respecté et que l'on contrôle le titre de l'acide sulfurique, en sortie dans le flux des acides résiduaires.

En fin de réaction de mononitration, on obtient une phase organique comprenant majoritairement du mononitrotoluène et plus précisément :
- de 75 à 97 % en poids de mononitrotoluène,
- de 2 à 15 % en poids de dinitrotoluène,
- de 1 à 10 % en poids d'acide nitrique,
- et moins de 1 % d'eau et d'acide sulfurique.

Conformément au procédé de l'invention, on effectue la nitration de la phase organique comprenant le mononitrotoluène, à l'aide d'acide nitrique en combinaison avec l'acide sulfurique. L'invention n'exclut pas la mise en oeuvre d'oléums. On peut donc faire appel aux oléums qui correspondent à l'acide sulfurique chargé d'anhydride sulfurique SO₃ dont la concentration peut varier entre 10 % et 40 % en poids. Des oléums à 20 % et à 40 % en poids de SO₃ sont commercialement disponibles.

On fait appel préférentiellement à un mélange nitrant à faible teneur en eau et plus particulièrement à un mélange nitrant comprenant de 55 à 70 % en poids d'acide sulfurique, de 20 à 44,5 % d'acide nitrique et de 0,5 à 10 % en poids d'eau.

D'une manière préférée, on choisit une teneur en eau comprise entre 0,5 et 9 % en poids.

Comme la mononitration, cette étape peut être conduite en discontinu mais préférentiellement selon un mode de réalisation en continu.

Ainsi dans un réacteur de nitration comme précédemment décrit, on introduit en parallèle, la phase organique et le mélange nitrant.

Deux paramètres sont pris en compte pour déterminer le débit des deux flux à savoir le ratio molaire acide nitrique/mononitrotoluène et la concentration d'acide sulfurique récupérée dans la phase aqueuse des acides résiduaires.

Le débit des deux flux est déterminé de telle sorte que le ratio molaire acide nitrique/mononitrotoluène soit compris entre 1,03 et 1,25.

Selon un mode de réalisation préféré de l'invention, la concentration d'acide nitrique dans le milieu peut être abaissée et le ratio molaire acide nitrique/mononitrotoluène choisi dans la partie basse de la fourchette c'est-à-dire choisi avantageusement entre 1,03 et 1,15.

Le débit des flux est également adapté de telle sorte que la teneur de l'acide sulfurique présent dans la phase aqueuse issue de la séparation soit comprise entre 70 et 90 %, de préférence entre 74 et 83,5 % en poids d'acide sulfurique exprimé par rapport au poids de tous les constituants y compris les composés organiques de la phase aqueuse.

Le procédé de l'invention est avantageusement conduit à une température comprise entre 50 et 90°C, de préférence entre 60°C et 80°C.

Il est à noter que la réaction de dinitration peut être conduite dans un réacteur ou bien dans une cascade de réacteurs, par exemple de 2 ou 3 réacteurs dont la température peut être égale ou bien différente : dans ce cas, généralement la température choisie dans l'intervalle précité, croit du premier réacteur jusqu'au dernier.

La réaction de nitration du mononitrotoluène est généralement mise en oeuvre sous pression atmosphérique bien que des pressions plus élevées peuvent être également utilisées.

Selon une variante préférée du procédé de l'invention, on conduit cette étape de nitration sous atmosphère contrôlée de gaz inertes afin d'être dans des conditions de concentration des produits gazeux dans le ciel en dehors de la zone d'inflammabilité. On établit préférentiellement une atmosphère d'azote.

En fin de réaction, on obtient une phase aqueuse et une phase organique qu'il faut séparer.

La séparation des phases aqueuse et organique est une opération qui peut être conduite par exemple dans une centrifugeuse ou un décanteur statique.

Toutefois, au préalable, conformément au procédé de l'invention, on effectue après l'opération de nitration du nitrotoluène et avant la séparation des phases organique et aqueuse, l'ajout de la phase aqueuse récupérée suite à la séparation des phases organique et aqueuse, de telle sorte que le rapport pondéral entre la phase aqueuse et la phase organique soit au moins égal à 1,2, de préférence compris entre 1,5 et 3,5, et encore plus préférentiellement compris entre 1,8 et 3,0.

On peut recycler la phase aqueuse récupérée suite à l'opération de séparation qui peut être conduite par exemple dans une centrifugeuse ou un décanteur statique.

On préfère recycler une fraction de la phase aqueuse récupérée, de préférence moins de 90 % en poids et encore plus préférentiellement, de 40 à 80 % en poids, l'autre fraction aqueuse acide pouvant être recyclée en amont du procédé, en particulier comme source d'acide sulfurique à l'étape de mononitration du toluène.

Le débit de recyclage de la phase aqueuse acide est calculé de telle sorte que le rapport pondéral entre la phase aqueuse et la phase organique soit au moins égal à 1,2, de préférence compris entre 1,5 et 3,5, et encore plus préférentiellement compris entre 1,8 et 3,0.

L'introduction du flux aqueux acide en amont de la séparation peut s'effectuer par exemple par l'intermédiaire d'un dispositif de mélange du flux principal biphasique et du flux aqueux acide, comme par exemple un mélangeur statique.

Ainsi, après recyclage de la phase aqueuse acide avant l'opération de séparation, la composition préférée des phases aqueuse et organique est la suivante :
- la phase aqueuse comprenant majoritairement de l'acide sulfurique a la composition suivante :
   - de 74 à 83,5 % en poids d'acide sulfurique,
   - de 0,5 à 3,5 % en poids d'acide nitrique,
   - de 8 à 18,5 % en poids d'eau,
   - de 4 à 8 % en poids de dinitrotoluène soluble.
- la phase organique comprenant majoritairement du dinitrotoluène a la composition suivante :
   - de 89,25 à 98,77 % en poids de dinitrotoluène,
   - de 1 à 7 % en poids d'acide nitrique,
   - de 0,1 à 2 % en poids d'acide sulfurique,
   - de 0,03 à 1,5 % en poids d'eau,
   - de 0,1 à 0,25 % en poids d'impuretés organiques (dinitrocrésols, trinitrocrésols, acide dinitrobenzoïque).

Selon une variante préférée du procédé de l'invention, la phase aqueuse comprend une concentration moindre d'acide nitrique qui se situe avantageusement entre 0,5 et 1,5 %.

Le procédé de l'invention est particulièrement intéressant car il permet de baisser les temps de séparation par décantation qui peuvent être de moins de 1 minute à 30 secondes.

Quand on passe d'un système de phase organique continue à un système de phase aqueuse continue, il est alors possible de diminuer l'excès d'acide nitrique mis en oeuvre lors de la deuxième réaction de nitration.

On donne ci-après un exemple d'installation dans lequel le procédé de l'invention peut être mis en oeuvre et qui comprend :
- un réacteur de nitration du mononitrotoluène équipé de moyens d'introduction des réactifs (mononitrotoluène, mélange nitrant), de moyens de chauffage, d'un système d'agitation et dans sa partie inférieure, d'un système de soutirage du mélange réactionnel,
- un deuxième réacteur de finition de la réaction de nitration relié par un conduit à la sortie du premier réacteur et équipé de moyens d'introduction du mélange réactionnel issu du premier réacteur et dans sa partie inférieure, d'un système de soutirage du mélange réactionnel,
- un dispositif de séparation des phases organique et aqueuse (de préférence décanteur, centrifugeuse) relié à la sortie du deuxième réacteur par un conduit,
- un moyen d'introduction d'une fraction de la phase aqueuse acide issue de l'opération de séparation des phases organique et aqueuse, soit au niveau du conduit reliant le deuxième réacteur de finition au dispositif de séparation des phases ou soit en amont du deuxième réacteur de finition sur le conduit reliant la sortie du réacteur de nitration à l'entrée du deuxième réacteur de finition ou directement à l'entrée du réacteur de finition.

Les différents appareillages sont reliés par des tuyauteries assurant la circulation et le recyclage des réactifs.

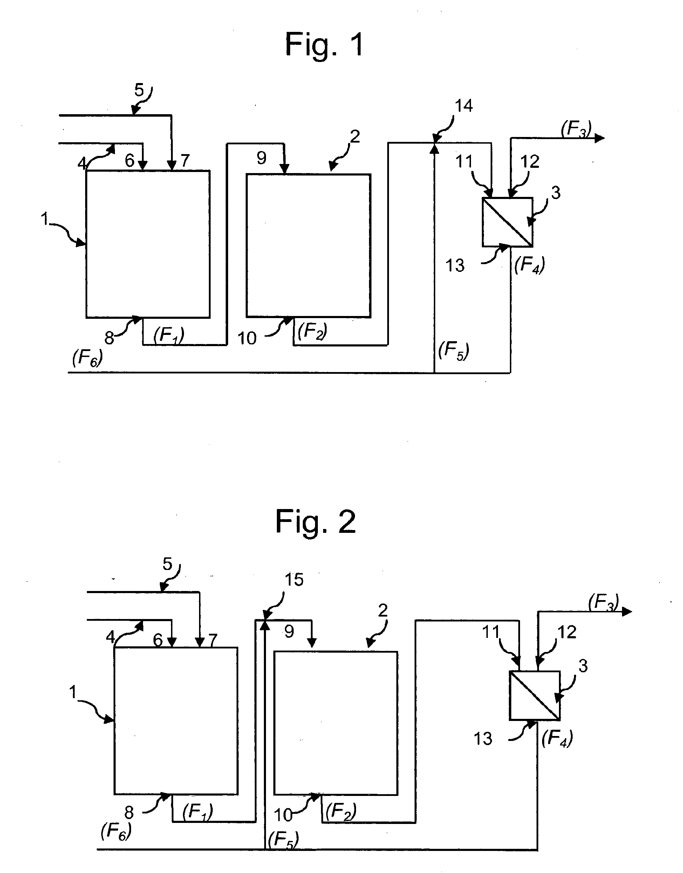

Deux modes de réalisation pratique de l'invention sont illustrés par les dessins annexés sous forme des figures 1 et 2.

Les figures 1 et 2 sont une représentation schématique des différents appareillages adaptés à la mise en oeuvre de l'invention et qui diffèrent l'un de l'autre, par le point d'introduction de la phase aqueuse acide.

L'ensemble décrit sur la figure 1 comprend un réacteur (1) de nitration du mononitrotoluène dans lequel sont introduits le mononitrotoluène (4) et le mélange nitrant (5) respectivement en (6) et (7).

Le mélange réactionnel *(F₁)* sortant en (8) du réacteur (1) est introduit en (9) dans un deuxième réacteur (2) dans lequel se finit la réaction de nitration.

En sortie en (10) du deuxième réacteur (2), on récupère un liquide bi-phasique (F₂) qui va être soumis à une opération de séparation qui sur la figure 1, est un décanteur (3) ce qui permet la séparation en (13) d'une phase aqueuse acide *(F₄)* et en (12) une phase organique *(F₃)* de dinitrotoluène qui peut être éventuellement envoyée à la purification.

La phase aqueuse acide *(F₄)* est fractionnée en deux parties, une fraction *(F₅)* et une fraction *(F₆)*.

La fraction *(F₅)* de la phase aqueuse acide est introduite par l'intermédiaire d'un dispositif approprié, par exemple un mélangeur statique ou un circulateur sur le conduit reliant le réacteur (2) et le décanteur (3), en un point d'alimentation (14) situé entre (10) et (11).

L'autre fraction *(F₆)* de phase aqueuse acide est recyclée en amont du procédé.

La figure 2 reproduit l'appareillage de la figure 1 à l'exception du flux *(F₅)* qui est recyclé non pas en amont du décanteur (3) mais par exemple par l'intermédiaire d'un branchement en un point (15) situé entre (8) et (9), en amont du deuxième réacteur (2) ou au niveau de l'entrée du réacteur (2).

L'invention va être maintenant décrite plus en détail à l'aide de modes de réalisation pris à titre d'exemples non limitatifs.

Dans les exemples, les abréviations ont la signification suivante :
- MNT : mononitrotoluène,
- DNT : dinitrotoluène,
- SN : mélange sulfonitrique,
- AS : acide sulfurique,
- AN : acide nitrique.

### Exemple comparatif 1

Un réacteur continu de nitration est alimenté par 772 kg/h de MNT brut contenant 6,1 % en poids de DNT et 91 % en poids de MNT (H₂SO₄ : 0,6 % en poids et HNO₃ 2,3 % en poids).

Est également coalimenté, à raison de 1067 kg/h, un mélange sulfonitrique fait à partir d'acide sulfurique 96 % et d'acide nitrique 99 % en poids.

La composition du SN est de AS : 62,8 % en poids, AN : 34 % en poids et de l'eau en quantité suffisante pour obtenir 100 %.

Le volume du réacteur est de 200 litres.

La température de réaction est de 65°C.

Les calories de la réaction sont évacuées par des échangeurs de chaleur.

L'excès d'acide nitrique par rapport au MNT est de 18 % mol.

En sortie du réacteur, les deux phases sont séparées par centrifugation.

Un échantillon est prélevé en amont de la centrifugeuse.

On détermine un temps de décantation voisin de 48 heures.

### Exemple comparatif 2

On reproduit l'essai précédent mais en baissant l'excès d'acide nitrique à 8 % molaire ce qui correspond à 1022 kg/h d'un mélange sulfonitrique à 65,5 % en poids d'AS.

On note que la séparation des phases devient alors impossible même par centrifugation, l'opération devant être arrêtée.

Un échantillon prélevé n'est pas totalement décanté au bout de plusieurs jours.

### Exemple 3

Un réacteur continu de nitration est alimenté par 772 kg/h de MNT brut contenant 6,1 % en poids de DNT et 91 % en poids de MNT (H₂SO₄ : 0,6 % en poids et HNO₃ 2,3 % en poids). Est également coalimenté, à raison de 1067 kg/h, un mélange sulfonitrique fait à partir d'acide sulfurique 96 % et d'acide nitrique 99 % en poids.

La composition du SN est de AS : 62,8 % en poids, AN: 34 % en poids et eau qsp 100 %.

L'excès d'acide nitrique par rapport au MNT est de 18 % en mol.

En sortie, les deux phases sont séparées par centrifugation.

Une partie de la phase acide (soit 65 % en poids) est recyclée comme selon la figure 1, en sortie du réacteur de finition, via un dispositif adapté de mélange (mélangeur statique) ou petite capacité agitée de 50 l dans notre cas.

Un échantillon de l'alimentation de la centrifugeuse est prélevé au bout d'une heure.

Le temps de décantation est de l'ordre de 30 secondes.

La partie non recyclée de l'acide résiduaire retourne vers la mononitration.

### Exemple 4

On reproduit l'exemple 3 à l'exception de l'excès d'acide nitrique qui est abaissé jusqu'à 6 % mol (contre un excès de 18 % mol dans l'exemple 3).

L'excès est contrôlé en mesurant l'acide nitrique dans l'acide résiduaire qui passe de 2 à moins de 1 % en poids.

Le temps de décantation de l'échantillon prélevé à l'alimentation de la centrifugeuse reste voisin de 30 secondes

Le procédé de l'invention qui réside dans un changement de phase continue (phase organique continue → phase acide continue) rendue possible par la mise en place du recyclage de la phase acide, permet de passer d'un temps de décantation mesuré à l'alimentation des centrifugeuses de 48 heures avec un excès d'acide nitrique fort (18 % mol) à un temps de décantation de 30 secondes avec un excès d'acide nitrique réduit (6 % mol).

## Revendications

1. Procédé de préparation de dinitrotoluène à partir du mononitrotoluène qui comprend la réaction de nitration du mononitrotoluène à l'aide d'un mélange nitrant comprenant de l'acide nitrique, de l'acide sulfurique et de l'eau conduisant à un milieu bi-phasique puis la séparation des phases organique et aqueuse dudit milieu bi-phasique, au cours duquel
∘ on effectue la nitration du mononitrotoluène à l'aide d'un mélange nitrant comprenant au plus 10 % en poids d'eau conduisant à un milieu bi-phasique,
∘ on sépare les phases organique et aqueuse dudit milieu bi-phasique, et
∘ on recycle la phase aqueuse issue de l'opération de séparation, en fin de réaction de nitration du mononitrotoluène et avant la séparation des phases organique et aqueuse de telle sorte que le rapport pondéral entre la phase aqueuse et la phase organique soit au moins égal à 1,2.

2. Procédé selon la revendication 1 **caractérisé par le fait que** le mélange nitrant comprend de 55 à 70 % en poids d'acide sulfurique, de 20 à 44,5 % d'acide nitrique et de 0,5 à 10 % en poids d'eau.

3. Procédé selon l'une des revendications 1 et 2 **caractérisé par le fait que** l'on introduit en parallèle, la phase organique comprenant le mononitrotoluène et le mélange nitrant.

4. Procédé selon la revendication 3 **caractérisé par le fait que** le débit des deux flux est déterminé de telle sorte que le ratio molaire acide nitrique/mononitrotoluène soit compris entre 1,03 et 1,25.

5. Procédé selon l'une des revendications 1 à 4 **caractérisé par le fait que** le débit des flux est également adapté de telle sorte que la teneur de l'acide sulfurique présent dans la phase aqueuse issue de la séparation soit comprise entre 70 et 90 %, de préférence entre 74 et 83,5 % en poids d'acide sulfurique exprimé par rapport au poids de tous les constituants y compris les composés organiques de la phase aqueuse.

6. Procédé selon la revendication 1 **caractérisé par le fait que** l'ajout de la phase aqueuse issue de l'opération de séparation des phases organique et aqueuse est fait de telle sorte que le rapport pondéral entre la phase aqueuse et la phase organique soit compris entre 1,5 et 3,5, et de préférence compris entre 1,8 et 3,0.

7. Procédé selon la revendication 1 **caractérisé par le fait que** l'on recycle la phase aqueuse séparée en fin de réaction de nitration du mononitrotoluène lorsque le taux de transformation du toluène est d'au moins 90 %.

8. Procédé selon l'une des revendications 1 à 7 **caractérisé par le fait que** l'introduction de la phase aqueuse acide en amont de la séparation est effectuée de préférence par l'intermédiaire d'un dispositif de mélange du flux principal biphasique et du flux aqueux acide, préférentiellement un mélangeur statique.

9. Procédé selon l'une des revendications 1 à 8 **caractérisé par le fait que** la phase aqueuse après recyclage de la phase aqueuse acide avant l'opération de séparation, comprenant majoritairement de l'acide sulfurique a la composition suivante :
. de 74 à 83,5 % en poids d'acide sulfurique,
. de 0,5 à 3,5 % en poids d'acide nitrique,
. de 8 à 18,5 % en poids d'eau,
. de 4 à 8 % en poids de dinitrotoluène soluble.

10. Procédé selon la revendication 9 **caractérisé par le fait que** la phase aqueuse comprend une concentration d'acide nitrique qui se situe entre 0,5 et 1,5 % en poids.

11. Procédé selon l'une des revendications 1 à 10 **caractérisé par le fait que** la phase organique après recyclage de la phase aqueuse acide avant l'opération de séparation, comprenant majoritairement du dinitrotoluène a la composition suivante :
. de 89,25 à 98,77 % en poids de dinitrotoluène,
. de 1 à 7 % en poids d'acide nitrique,
. de 0,1 à 2 % en poids d'acide sulfurique,
. de 0,03 à 1,5 % en poids d'eau,
. de 0,1 à 0,25 % en poids d'impuretés organiques (dinitrocrésols, trinitrocrésols, acide dinitrobenzoïque).

12. Procédé selon l'une des revendications 1 à 11 **caractérisé par le fait que** la phase organique comprenant le mononitroluène est préparé par nitration du toluène avec l'acide nitrique, en présence d'acide sulfurique ce qui conduit à un milieu bi-phasique suivie d'une séparation des phases aqueuse et organique.

13. Procédé selon l'une des revendications 1 à 12 **caractérisé par le fait que** le dinitrotoluène est un mélange d'isomères de 2,4-dinitrotoluène et de 2,6-dinitrotoluène.

14. Procédé selon la revendication 13 **caractérisé par le fait que** le ratio entre le 2,4-dinitrotoluène et le 2,6-dinitrotoluène est égal à 4 ± 0,3 et la teneur en impuretés est comprise entre 3 et 4,5 % en poids.

15. Utilisation d'un dispositif qui comprend :
- un réacteur de nitration (1) du mononitrotoluène équipé de moyens d'introduction des réactifs (mononitrotoluène, mélange nitrant), de moyens de chauffage, d'un système d'agitation et dans sa partie inférieure, d'un système de soutirage du mélange réactionnel,
- un deuxième réacteur (2) de finition de la réaction de nitration relié par un conduit à la sortie du premier réacteur et équipé de moyens d'introduction du mélange réactionnel issu du premier réacteur et dans sa partie inférieure, d'un système de soutirage du mélange réactionnel,
- un dispositif de séparation (3) des phases organique et aqueuse (de préférence décanteur, centrifugeuse) relié à la sortie du deuxième réacteur par un conduit,
- un moyen d'introduction d'une fraction de la phase aqueuse acide issue de l'opération de séparation des phases organique et aqueuse, soit au niveau du conduit reliant le deuxième réacteur (2) de finition au dispositif de séparation (3) des phases ou soit en amont du deuxième réacteur (2) de finition sur le conduit reliant la sortie du réacteur de nitration (1) à l'entrée du deuxième réacteur (2) de finition ou directement à l'entrée du réacteur de finition (2),
pour la mise en oeuvre du procédé de l'une des revendications 1 à 14.

## Patentansprüche

1. Verfahren zur Herstellung von Dinitrotoluol aus Mononitrotoluol, das die Nitrierungsreaktion des Mononitrotoluols mittels eines Nitriergemischs, das Salpetersäure, Schwefelsäure und Wasser umfasst, was zu einem zweiphasigen Medium führt, dann die Trennung der organischen und wässrigen Phase des zweiphasigen Mediums umfasst, in dessen Verlauf
∘ die Nitrierung des Mononitrotoluols mittels eines Nitriergemischs durchgeführt wird, das höchstens 10 Gew.-% Wasser umfasst, was zu einem zweiphasigen Medium führt,
∘ die organische und wässrige Phase des zweiphasigen Mediums getrennt werden, und
∘ die sich aus dem Trennvorgang ergebende wässrige Phase am Ende der Nitrierungsreaktion des Mononitrotoluols und vor der Trennung der organischen und wässrigen Phase so rezykliert wird, dass das Gewichtsverhältnis zwischen der wässrigen Phase und der organischen Phase mindestens gleich 1,2 ist.

2. Verfahren nach Anspruch 1, **dadurch gekennzeichnet, dass** das Nitriergemisch 55 bis 70 Gew.-% Schwefelsäure, 20 bis 44,5 Gew.-% Salpetersäure und 0,5 bis 10 Gew.-% Wasser umfasst.

3. Verfahren nach einem der Ansprüche 1 und 2, **dadurch gekennzeichnet, dass** die das Mononitrotoluol umfassende organische Phase und das Nitriergemisch parallel eingebracht werden.

4. Verfahren nach Anspruch 3, **dadurch gekennzeichnet, dass** der Durchfluss der zwei Ströme so bestimmt wird, dass das molare Verhältnis Salpetersäure/Mono-nitrotoluol zwischen 1,03 und 1,25 beträgt.

5. Verfahren nach einem der Ansprüche 1 bis 4, **dadurch gekennzeichnet, dass** der Durchfluss der Ströme auch so angepasst wird, dass der Gehalt an Schwefelsäure, die in der sich aus der Trennung ergebenden wässrigen Phase vorhanden ist, zwischen 70 und 90%, vorzugsweise zwischen 74 und 83,5% des Schwefelsäuregewichts ausgedrückt durch das Gewichtsverhältnis aller Bestandteile einschließlich der organischen Verbindungen der wässrigen Phase beträgt.

6. Verfahren nach Anspruch 1, **dadurch gekennzeichnet, dass** die Zugabe der wässrigen Phase, die sich aus dem Trennvorgang der organischen und wässrigen Phase ergibt, so erfolgt, dass das Gewichtsverhältnis zwischen der wässrigen Phase und der organischen Phase zwischen 1,5 und 3,5 und vorzugsweise zwischen 1,8 und 3,0 beträgt.

7. Verfahren nach Anspruch 1, **dadurch gekennzeichnet, dass** die abgetrennte wässrige Phase am Ende der Nitrierungsreaktion des Mononitrotoluols rezykliert wird, wenn der Umwandlungsgrad des Toluols mindestens 90% beträgt.

8. Verfahren nach einem der Ansprüche 1 bis 7, **dadurch gekennzeichnet, dass** die Einbringung der sauren wässrigen Phase vor der Trennung vorzugsweise mittels einer Mischvorrichtung für den zweiphasigen Hauptstrom und den sauren wässrigen Strom, bevorzugt mittels eines statischen Mischers erfolgt.

9. Verfahren nach einem der Ansprüche 1 bis 8, **dadurch gekennzeichnet, dass** die wässrige Phase nach der Rezyklierung der sauren wässrigen Phase vor dem Trennvorgang, die mehrheitlich Schwefelsäure umfasst, die folgende Zusammensetzung hat:
- 74 bis 83,5 Ges.-% Schwefelsäure,
- 0,5 bis 3,5 Ges.-% Salpetersäure,
- 8 bis 18,5 Gew.-% Wasser,
- 4 bis 8 Gew.-% lösliches Dinitrotoluol.

10. Verfahren nach Anspruch 9, **dadurch gekennzeichnet, dass** die wässrige Phase eine Salpetersäurekonzentration hat, die sich zwischen 0,5 und 1,5 Gew.-% befindet.

11. Verfahren nach einem der Ansprüche 1 bis 10, **dadurch gekennzeichnet, dass** die organische Phase nach der Rezyklierung der sauren wässrigen Phase vor dem Trennvorgang, die mehrheitlich Dinitrotoluol umfasst, die folgende Zusammensetzung hat:
- 89,25 bis 98,77 Gew.-% Dinitrotoluol,
- 1 bis 7 Gew.-% Salpetersäure,
- 0,1 bis 2 Gew.-% Schwefelsäure,
- 0,03 bis 1,5 Gew.-% Wasser,
- 0,1 bis 0,25 Gew.-% organische Verunreinigungen (Dinitrokresole, Trinitrokresole, Dinitrobenzoesäure).

12. Verfahren nach einem der Ansprüche 1 bis 11, **dadurch gekennzeichnet, dass** die organische Phase, die das Mononitrotoluol umfasst, durch Nitrierung des Toluols mit Salpetersäure im Beisein von Schwefelsäure, was zu einem zweiphasigen Medium führt, gefolgt von einer Trennung der wässrigen und organischen Phase hergestellt wird.

13. Verfahren nach einem der Ansprüche 1 bis 12, **dadurch gekennzeichnet, dass** das Dinitrotoluol ein Isomerengemisch aus 2,4-Dinitrotoluol und 2,6-Dinitrotoluol ist.

14. Verfahren nach Anspruch 13, **dadurch gekennzeichnet, dass** das Verhältnis zwischen dem 2,4-Dinitrotoluol und dem 2,6-Dinitrotoluol gleich 4 ±3 ist, und der Gehalt an Verunreinigungen zwischen 3 und 4,5 Ges.-% beträgt.

15. Verwendung einer Vorrichtung, die umfasst:
- einen Nitrierungsreaktor (1) für Mononitrotoluol, der mit Einbringungseinrichtungen für Reagenzien (Mononitrotoluol, Nitriergemisch), Erwärmungseinrichtungen, einem Rührsystem und in seinem unteren Teil einem Entnahmesystem für das Reaktionsgemisch ausgestattet ist,
- einen zweiten Reaktor (2) zur Fertigstellung der Nitrierungsreaktion, der über eine Leitung an den Ausgang des ersten Reaktors angeschlossen und mit Einbringungseinrichtungen für das sich aus dem ersten Reaktor ergebende Reaktionsgemisch und in seinem unteren Teil mit einem Entnahmesystem für das Reaktionsgemisch ausgestattet ist,
- eine Trennvorrichtung (3) für die organische und wässrige Phase (vorzugsweise Abscheider, Zentrifuge), die über eine Leitung an den Ausgang des zweiten Reaktors angeschlossen ist,
- eine Einrichtung zum Einleiten einer Fraktion der sauren wässrigen Phase, die sich aus dem Trennvorgang der organischen und wässrigen Phase ergeben hat, entweder im Bereich der Leitung, die den zweiten Fertigstellungsreaktor (2) mit der Trennvorrichtung (3) für die Phasen verbindet, oder vor dem zweiten Fertigstellungsreaktor (2) über die Leitung, die den Ausgang des Nitrierungsreaktors (1) mit dem Eingang des zweiten Fertigstellungsreaktors (2) verbindet, oder direkt am Eingang des Fertigstellungsreaktors (2),
zur Umsetzung des Verfahrens nach einem der Ansprüche 1 bis 14.

## Claims

1. Method for preparing dinitrotoluene from mononitrotoluene comprising the nitration reaction of mononitrotoluene using a nitrating admixture comprising nitric acid, sulphuric acid and water, and resulting in a bi-phase medium, then separation of the organic and aqueous phases of the bi-phase medium, wherein
∘ nitration of the mononitrotoluene is carried out using a nitrating admixture comprising a maximum of 10 % by weight of water resulting in a bi-phase medium,
∘ separating the organic and aqueous phases of the bi-phase medium, and
∘ recycling the aqueous phase from the separation operation, at the end of the nitration reaction of the mononitrotoluene and before separating the organic and aqueous phases, such that the weight ratio between the aqueous phase and the organic phase is at least 12.

2. Method according to claim 1, **characterised in that** the nitrating admixture comprises from 55 to 70 % by weight of sulphuric acid, from 20 to 44.5 % of nitric acid and from 0.5 to 10 % by weight of water.

3. Method according to either claim 1 or claim 2, **characterised in that** the organic phase comprising the mononitrotoluene and the nitrating admixture are introduced in parallel.

4. Method according to claim 3, **characterised in that** the flow rate of the two flows is determined so that the molar ratio of nitric acid/mononitrotoluene is between 1.03 and 1.25.

5. Method according to any one of claims 1 to 4, **characterised in that** the flow rate of the flows is also adapted so that the content of sulphuric acid present in the aqueous phase resulting from the separation, is between 70 and 90 %, preferably between 74 and 83.5 % by weight of sulphuric acid expressed relative to the weight of all the constituents including the organic compounds of the aqueous phase.

6. Method according to claim 1, **characterised in that** the addition of the aqueous phase resulting from the separation operation of the organic and aqueous phases, is carried out so that the weight ratio between the aqueous phase and the organic phase is between 1.5 and 3.5, and preferably between 1.8 and 3.0.

7. Method according to claim 1, **characterised in that** the separated aqueous phase is recycled at the end of the nitration reaction of the mononitrotoluene when the conversion level of toluene is at least 90 %.

8. Method according to any one of claims 1 to 7, **characterised in that** the introduction of the acid aqueous phase upstream of the separation, is preferably carried out using a device for mixing the main bi-phase flow and the acid aqueous flow, preferably a static mixer.

9. Method according to any one of claims 1 to 8, **characterised in that** the aqueous phase, after recycling the acid aqueous phase before the separation operation, mainly comprising sulphuric acid, has the following composition:
- from 74 to 83.5 % by weight of sulphuric acid,
- from 0.5 to 3.5 % by weight of nitric acid,
- from 8 to 18.5 % by weight of water,
- from 4 to 8 % by weight of soluble dinitrotoluene.

10. Method according to claim 9, **characterised in that** the aqueous phase comprises a concentration of nitric acid that is between 0,5 and 1.5 % by weight.

11. Method according to any one of claims 1 to 10, **characterised in that** the organic phase, after recycling of the acid aqueous phase, before the separation operation, mainly comprising dinitrotoluene, has the following composition:
- from 89.25 % to 98.77 % by weight of dinitrotoluene,
- from 1 to 7 % by weight of nitric acid,
- from 0.1 to 2 % by weight of sulphuric acid,
- from 0.03 to 1.5 % by weight of water,
- from 0.1 to 0.25 % by weight of organic impurities (dinitrocresols, trinitrocresols, dinitrobenzoic acid).

12. Method according to any one of claims 1 to 11, **characterised in that** the organic phase comprising mononitrotoluene is prepared by nitrating toluene with the nitric acid, in the presence of sulphuric acid, resulting in a bi-phase medium, followed by separating the aqueous and organic phases.

13. Method according to any one of claims 1 to 12, **characterised in that** dinitrotoluene is an admixture of isomers of 2,4-dinitrotoluene and 2,6-dinitrotoluene.

14. Method according to claim 13, **characterised in that** the ratio between 2,4-dinitrotoluene and 2,6-dinitrotoluene is equal to 4 ± 0.3 and the content in impurities is between 3 and 4.5 % by weight.

15. Use of an apparatus comprising:
∘ a reactor (1) for nitration of mononitrotoluene provided with means for introducing the reagents (mononitrotoluene, nitrating admixture), heating means, a stirring system and, in the lower portion thereof, a system for tapping the reaction mixture,
∘ a second reactor (2) for finishing the nitration reaction, connected by a pipe to the outlet of the first reactor and provided with means for introducing the reaction admixture from the first reactor and, in the lower portion thereof, a system for tapping the reaction admixture,
∘ a device (3) for separating the organic and aqueous phases (preferably a decanter, a centrifuge) connected to the outlet of the second reactor by means of a pipe,
∘ a mean for introducing a fraction of the acid aqueous phase resulting from the separation operation of the organic and aqueous phases, either in the region of the pipe that connects the second finishing reactor (2) to the phase separation device (3), or upstream of the second finishing reactor (2) on the pipe connecting the outlet of the nitration reactor (1) to the inlet of the second finishing reactor (2) or directly to the inlet of the finishing reactor (2),
for implementing the method of any one of claims 1 to 14.
